Europäisches Patentamt

European Patent Office

Office européen des brevets

(19)

(11) Publication number: **0 686 397 A2**

# EUROPEAN PATENT APPLICATION

(12)

(21) Application number: 95106534.1

(22) Date of filing: 29.04.95

(51) Int. Cl.⁶: **A61K 38/17**, C07K 14/47, C12P 21/02

(30) Priority: 09.05.94 PCT/EP94/01479

(43) Date of publication of application:
**13.12.95 Bulletin 95/50**

(84) Designated Contracting States:
**AT BE CH DE DK ES FR GB GR IE IT LI LU NL PT SE**

(71) Applicant: **BOEHRINGER MANNHEIM GMBH**
**Sandhofer Strasse 116**
**D-68305 Mannheim (DE)**

(72) Inventor: **Müller, Hans Werner, Prof.**
**Jakop-Kneip-Strasse 88**
**D-40595 Düsseldorf (DE)**

Inventor: **Junghans,Ulrich, Dr.**
**Karlstrasse 2**
**D-42699 Solingen (DE)**
Inventor: **Kappler, Joachim Dr.**
**Schumannstrasse 49**
**D-53113 Bonn (DE)**
Inventor: **Koops, Antje**
**Düsselthaler Strasse 41**
**D-40211 Düsseldorf (DE)**
Inventor: **Hasenöhrl, Rüdiger, Dr.**
**Sadowastrasse 29**
**D-42115 Wuppertal (DE)**
Inventor: **Huston, Joseph, Prof.**
**Graf-Recke-Strasse 70**
**D-40239 Düsseldorf (DE)**

(54) Use of chondroitin sulphate proteoglycans for protection of neurons

(57) The use of a proteoglycan with a part or all of the primary amino acid structure of SEQ ID NO:1 and at least with one attached glycosaminoglycan chains promotes neurite outgrowth, supports survival of neurons in vivo and in vitro and/or causes therapeutic effects in the regeneration of nervous cells.

**Fig. 11**

EP 0 686 397 A2

The invention is directed to the use of chondroitin sulphate proteoglycans for promotion of neurite outgrowth and support of survival of neurons. The invention also concerns methods and means for preparing these substances and pharmaceutical compositions comprising them.

Proteoglycans are proteins that carry one or more glycosaminoglycan side chains. Proteoglycans are found in a great variety in tissue, e.g. vascular smooth muscle and bone/cartilage. The glycosaminoglycan substitution depends on the ability of the protein to serve as an acceptor for xylosyl transferase, the enzyme that begins the synthesis of most types of glycosaminoglycans. Proteoglycans come in all sizes and shapes, and their only common feature may be the presence of the glycosaminoglycan. In this regard, a glycosaminoglycan substitution is similar to other types of glycosylation of proteins. However, it does not necessarily imply functional similarity. A review on the contribution of proteoglycans in cell regulation is given by Ruoslahti (1989 and 1991).

Proteins which regulate the proliferation and survival of neurons and their morphological plasticity are named neurotrophic factors. Such neurotrophic factors regulate also the synthesis of other neuron-specific proteins. A non-regular expression of neurotrophic factors in vivo can result in developmental disturbances and/or neurodegenerative diseases of the nervous system. Besides neurite promoting factors, diffusible neurotrophic activity and a critical cell density are essential requirements for prolonged survival of central nervous system neurons in culture (Müller et al., 1991). Some neurotrophic factors are proteoglycans. Schulz et al. (1990) purified a neurotrophic proteoglycan from superior colliculus with survival activity for chick retinal ganglion cells. Laminin-proteoglycan complexes and fibronectin were found to contribute predominantly to the neuritogenic activity (Matthiessen et al., 1991b).

However, the functions of most brain proteoglycans still remain unknown. Based on studies of connective tissue proteoglycans, it has been speculated that these molecules participate in the regulation of important cell functions like, e.g., cell growth and adhesion by modulating cell-cell and cell-substrate interactions, activation of growth factors and organization of the extracellular matrix (for review Ruoslahti, 1989; Ruoslahti and Yamaguchi, 1991).

The inventors have surprisingly found that a chondroitin sulphate proteoglycan from bone (also named hereinafter biglycan) promotes neurite outgrowth and supports survival of cultured CNS neurons. It was also found that in addition to the neurotrophic effect in vitro, the biglycan has a facilitatory effect on learning in vivo.

The isolation and idenfication of the neurotrophic activity as biglycan was possible according to the invention from meningeal fibroblasts under denaturing conditions preferably using an anion exchange chromatography with an optional additional molecular weight separation. It is especially preferred to use a three-step purification procedure: (i) initial anion exchange chromatography; (ii) rechromatography on an anion exchanger in the presence of a denaturation agent (preferably 8 M urea); and (iii) final gel filtration (e.g. on Superose 6) in the presence of a denaturing agent (preferably 4 M guanidinium hydrochloride).

Accordingly, the invention relates to the use of a proteoglycan with a part or all of the primary amino acid structure of SEQ ID NO: 1 and attached glycosaminoglycan chains for promotion of neurite outgrowth and support of survival of neurons in vivo and in vitro. In addition, the invention relates to the use of this proteoglycan for the manufacturing of a therapeutic agent for the prevention of neural cell degeneration and/or for the regeneration of the nervous system.

The term "neuron" refers to nerve cells which receive conduct and transmit signals. The significance of the signals varies according to the part played by the individual cell in the function of the nervous system as a whole. In a motor neuron, the signals represent commands for the contraction of a particular muscle. In a sensory neuron, they represent the information that a specific type of stimulus, such as light, a mechanical force or a chemical substance, is present at a certain site of the body. In an interneuron, they represent parts of a computation that combines sensory information from many different sources and generates an appropriate set of motor commands in response. Typically, three major portions of the neuron are distinguishable: The cell body, the dendrites, and the axon.

The term "nervous system" refers to the nervous system of a vertrebrate. It consists of brain, spinal cord and peripheral nerves. The nerve cell bodies lie either inside the central nervous system, or outside it is clustered in ganglia. In the central nervous system, the vast majority of the nerve cells are interneurons which both receive their inputs from, and deliver their output to, other nerve cells.

The term "neurite outgrowth" refers to the development of axons and dendrites from a growth cone. In principal, the neurite could grow by insertion of new material at its base, at its tip, or along its length.

The term "regeneration of the nervous system" means regeneration, especially of nerve cells, by development or elongation of axons and dendrites from a neuron and/or a growth cone.

The term "neural cell degeneration" means all processes which impair the receiving, conduction and transmitting of signals in the nervous system.

2

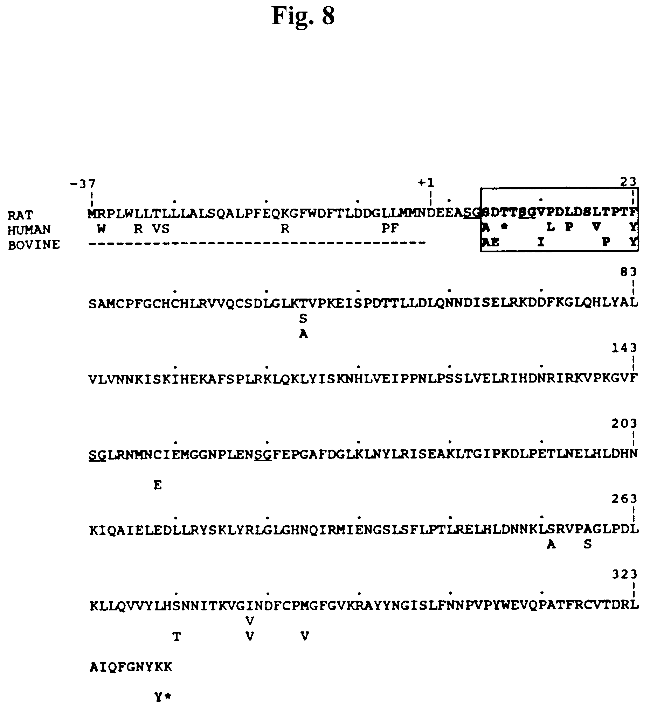

The protein which can be used according to the invention is a proteoglycan which is based on the DNA and amino acid sequence of SEQ ID NO:1. The corresponding human nucleotide sequence has been submitted to the GenBank™/EMBL Data Bank with Accession Number J 04599 by the authors of Fischer et al., J. Biol. Chem. 264 (1989) 4571-4576, incorporated herein by reference. The biglycan which can be used according to the invention may be derived from all mammalian species, whereby human, rat or bovine biglycan is preferred. The differences in the protein sequence of these three biglycans are shown in Fig. 8. It was found that between amino acid 7 - 23, a hypervariable region is included (Data according to Dreher et al., Eur. J. Cell Biol. 53 (1990) 296-304), incorporated herein by reference. So, this region could be altered considerably without altering the function according to the invention.

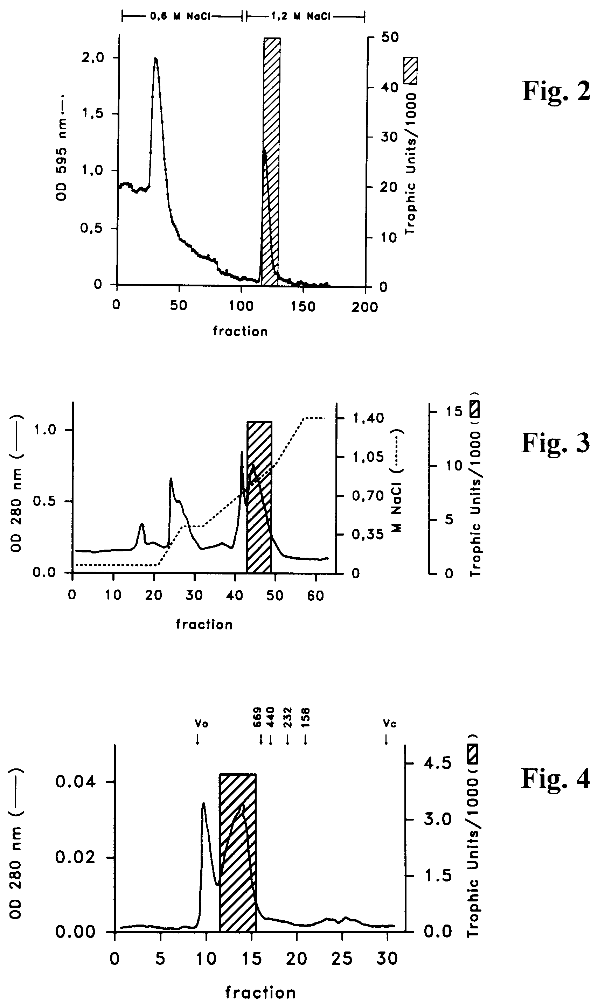

Naturally occurring biglycan has a molecular weight of about 42,500 for the complete sequence and about 38,000 for the secreted form. It consists predominantly of a series of 12 tandem repeats of 24 amino acid residues. The repeats are recognized by their conserved leucines and leucine-like amino acids (Fisher et al., J. Biol. Chemistry 264 (1989) 4571-4576, especially fig. 4, incorporated herein by reference).

Also other variations of the protein sequence of biglycan as long as the sequence is substantially identical with SEQ ID NO:1 are possible and as long as the function according to the invention is maintained. The sequence of SEQ ID NO:1 with or without the variations of Fig. 8 is preferred, however.

For example, for an improvement of the manufacturing of the vectors or optimizing of the expression, some modifications of the nucleic acid sequence are preferred. Such modifications are, for example:

alteration of the nucleic acid sequence for insertion of different recognition sequences for restriction enzymes for the improvement of ligation, cloning and mutagenesis;

alteration of the nucleic acid sequence for insertion of codons which are preferred by the host cell;

supplementation of the nucleic acid sequence for additional operator elements for optimizing the expression in the host cell.

Variants of biglycan which are useful according to the invention will possess at least 80 % homology, and preferably 90 % homology with SEQ ID NO:1 and/or Fig. 8.

Biglycan can be produced according to the methods known in the art, e.g. by isolation from natural material (e.g. meningeal fibroblasts, vascular smooth muscle or cartilage) or by recombinant technology. Such methods are described by Dreher (1990, supra), Fisher (1987, 1989, 1991), Neame et al., J. Biol. Chem. 264 (1989) 8653-8661, Rosenberg et al., J. Biol. Chem. 260 (1985) 6304-6313, and Stöcker et al., Biochem. J. 274 (1991) 415-420, the disclosures of which are hereby incorporated by reference.

It is preferred to produce the biglycan by recombinant methods. For this, the DNA sequence is cloned in a vector and transformed in a host cell for replication. Such a vector contains, in addition, operator elements for the expression of the DNA sequence.

The host cell is cultivated under conditions for the amplification of the vector, and after the fermentation, the desired protein is isolated. In this case, it is also possible to vary the glycosylation pattern of the protein.

It is preferred to produce the biglycan in a eukaryotic cell, as in such cells the glycosylation can occur in such a way that the biglycan with at least two attached glycosaminoglycan chains is produced.

However, it is also possible to attach the glycosaminoglycan side chain or chains after the expression (e.g. in prokaryotes such as E. coli) by methods according to the state of the art. Chemical and enzymatic coupling of glycosides to proteins can be accomplished using a variety of activated groups, for example as described by Alpin and Wriston in CRC Crit. Rev. Biochem. (1981) 259-306. This method is especially preferred if it is intended to produce a protein with one glycosaminoglycan side chain.

The modification to delete, change or insert appropriate amino acid(s) in the biglycan molecule to effect desired sequence variations is accomplished by any means known in the art, such as, e.g., site-directed mutagenesis or ligation of the appropriate sequence into the DNA encoding the relevant protein. Such methods are described, for example, in Sambrook et al., Molecular Cloning: A Laboratory Manual, 2nd edition, Cold Spring Harbor Laboratory Press, New York (1989).

PCR mutagenesis is also suitable for making a nucleic acid which codes for a biglycan of the present invention, for example as described in Current Protocols in Molecular Biology, Ausubel et al., edit. Screen Publishing Associates and Wiley Interscience, Vol. 2, Chapter 15 (1991).

The nucleic acid sequence (especially the cDNA) encoding the biglycan and its variants is inserted into a replicable vector for further cloning or expression. Suitable vectors are prepared using standard recombinant DNA procedures. Isolated plasmids and DNA fragments are cleaved, tailored and ligated together in a specific order to generate the desired vectors.

As already mentioned, cell cultures derived from eukaryotic (multicellular) organisms are preferred as hosts for the expression. While both invertebrate and vertebrate cell cultures are acceptable, vertebrate cell cultures, in particular mammalian cultures, are preferred. Examples of suitable cell lines include COS-7 (ATCC CRL1651), BHK (ATCC CCL10), Chinese hamster ovaria cells (CHO), monkey kidney cells (ATCC CCL70), Hela cells (ATCC CCL2), human lung cells (W138, ATCC CCL75), human liver cells (HepG2, HB8065), rat hepatoma cells (HTC, MI.54, Baumann et al., J. Cell Biol. 85 (1980) 1), whereby human cell lines are preferred.

As promoters there are preferred viral promoters as promoters derived from polyoma virus, Adenovirus 2 and Simian Virus 40 (SV40). Alternatively, promoters homologous to the biglycan and/or host cell line may also be useful. For enhancing the production levels, it is preferred to use on the vector also an amplifiable sequence, such as, e.g., the DHFR gene.

The compounds of the present invention can be formulated according to known methods to prepare pharmaceutically useful compositions, whereby the biglycan is combined in admixture with a pharmaceutically acceptable carrier. Such compositions will typically contain an effective amount of biglycan, together with a suitable amount of carrier to prepare a pharmaceutically acceptable composition suitable for effective administration to the patient. The biglycan may be administered parenterally to the patients, or by other methods which ensue its delivery to the central and peripheral nervous system.

The compositions include sterile aqueous solutions or lyophilisates; preferably with an appropriate amount of a pharmaceutically acceptable salt, to render the formulation isotonic, and/or a buffer to maintain a suitable pH, generally from 5 to 9.

Dosages and desired drug concentrations of the pharmaceutical compositions of this invention may vary depending on the used biglycan or its variant. The determination of the appropriate dosage is well within the skill of a physician.

Due to the neurotrophic activity of the biglycan, a therapeutic agent could be manufactured with the potential to enhance the survival and maintain the structure and function of CNS (central nervous system) neurons during normal ageing (this is usually accompanied by progressive nervous cell death) as well as after pathological and/or traumatic nervous system damage.

Due to the neurite growth-promoting activity of the biglycan, a therapeutic agent could be manufactured with the potential to stimulate sprouting and regeneration of damaged nervous fibers that may lead to restoration of function following nervous system lesions and degenerative diseases.

Due to the memory-enhancing activity of the biglycan, a therapeutic agent could be manufactured with the potential to improve learning efficiency and attenuate memory deficits in the elderly and patients with dementia.

The following figures, sequences and examples describe further details of the invention.


**Figure Legends**


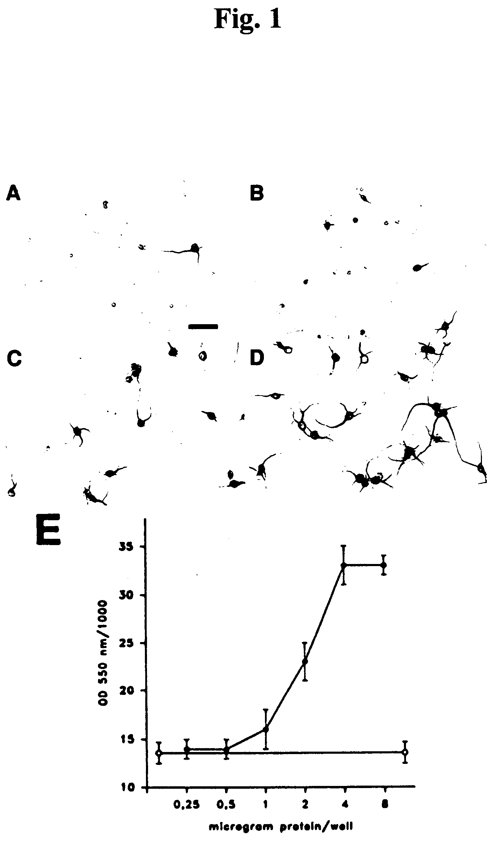

**Figure 1. Dosage dependent neurotrophic survival activity of MCM.** Neocortical neurons from embryonic rats (day E15) were cultured for 5 days in N2 (A, open circles in E) or in serial dilutions of MCM (B-D, filled circles in E) and either immunolabeled with anti-map2 antibodies (A-D) or stained with the vital dye MTT (E) as described in Materials and Methods of Example 1. Protein concentrations used in (B), (C), and (D) were 5, 10, and 20 $\mu$g/ml (corresponding to 1, 2 and 4 $\mu$g/well), respectively. Values in (E) represent mean ± s.d. of quadruplicates. Scale bar 50 $\mu$m.

**Figure 2. Chromatography of MCM on Q-Sepharose.** The first purification step was the anion-exchange chromatography of MCM in 50 mM Tris/HCl pH 7.4. Fractions eluted with a two-step salt gradient were assayed for protein concentration and neurotrophic activity (see Materials and Methods of Example 1). Biologically active fractions corresponding to the hatched bar were pooled (Q-Sepharose-Pool) for further purification.

**Figure 3. Chromatography of active Q-Sepharose-Pool on a MonoQ column in the presence of 8 M urea.** Q-Sepharose-Pool (see Fig. 2) was rechromatographed on MonoQ in 50 mM sodium acetate buffer (pH 6.0) containing 8 M urea, and protein was eluted with a linear salt gradient ranging from 0.07 to 1.4 M sodium chloride. The neurotrophic activity was eluted at high salt concentrations and active fractions (hatched bar) were pooled (MonoQ-Pool) for further purification.

**Figure 4. Gel filtration of MonoQ-Pool on Superose 6 in the presence of 4 M guanidinium hydrochloride.** The Superose 6 column was equilibrated with 50 mM Tris/HCl buffer (pH 7.4) containing 4 M guanidinium hydrochloride. Active material (hatched bar) was eluted at a Kav of 0.21. Calibration standards used were thyroglobin (669 kDa), ferritin (440 kDa), catalase (232 kDa) and aldolase (158 kDa), respectively. V0 (elution of blue dextran) was at 7.2 ml and Vc at 24 ml.

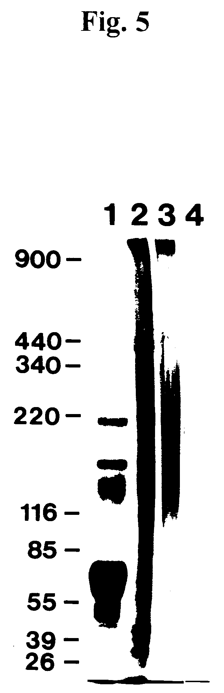

**Figure 5. Silver stained SDS-polyacrylamide gel electrophoresis of biologically active fractions after different purification steps.** Equal amounts of protein (5 $\mu$g each) of MCM (lane 1) and active fractions pooled after chromatography on Q-Sepharose (lane 2), MonoQ (lane 3) and Superose 6 (lane 4), respectively, were separated under reducing conditions on a 3-10 % SDS-gradient gel and visualized by silver staining. Numbers on the left indicate molecular weight standards in kDa.

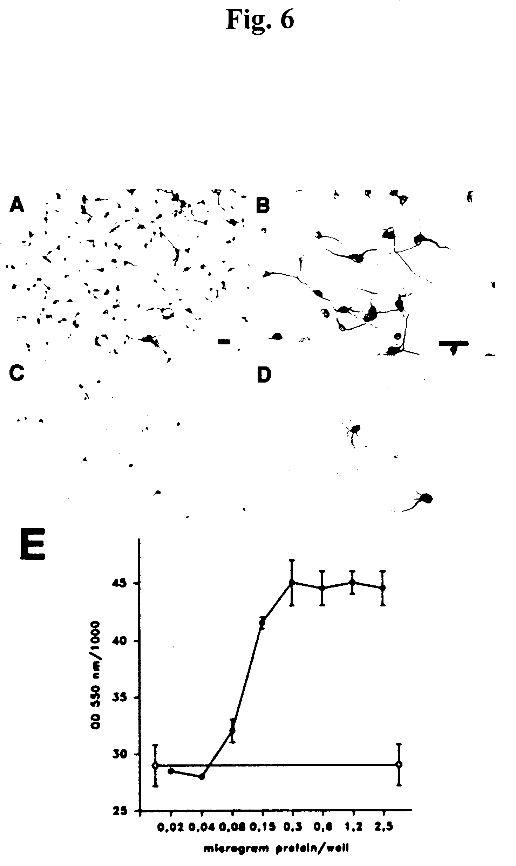

**Figure 6. Effect of purified biglycan from meningeal cells on the survival of embryonic rat neocortical neurons.** Neurons were cultured for 5 days in N2 medium supplemented with 1.5 $\mu$g purified biglycan/ml (A, B, filled circles in E) or in N2 medium alone (C, D, open circles in E) and either immunolabeled with anti-map2 antibodies (A-D) or stained with the vital dye MTT (E). Note the formation of cytoplasmic vacuoles and aberrant neuritic processes indicating cell degeneration leading to progressive neuronal death in N2 (D), while neurons cultured in presence of biglycan are viable and connected through a dense neuritic network (B). (E) illustrates the dose dependent activity of biglycan. Values in (E) represent mean ± SD of quadruplicates. Scale bars 50 $\mu$m (A, C and B,D, respectively).

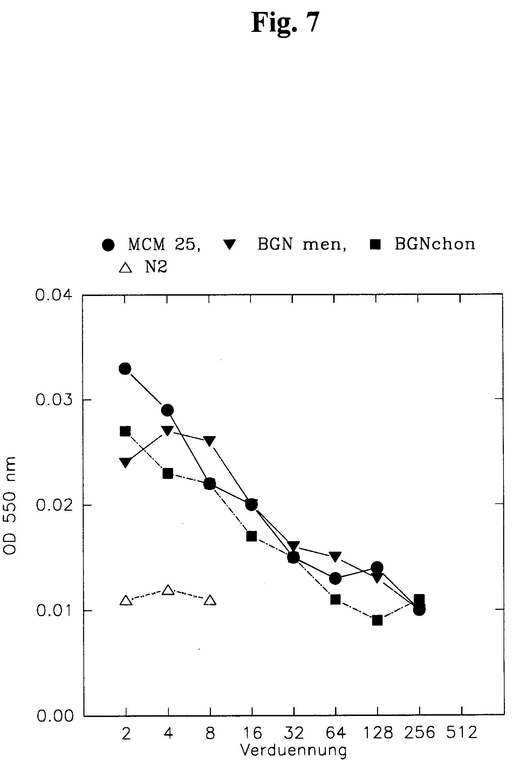

**Figure 7** describes in accordance with Fig. 6 E dose-response curves for rat biglycan (BGN-men), bovine biglycan (BGN-chon), culture supernatants of meningeal rat cells (MCM 25), and inactive culture medium (N2) as control. BGN-men and BGN-chon were used at the same initial concentration (0.02 $\mu$g biglycan/well of microtiter plate) (*OD550 nm*: optical density at 550 nm, *Verdünnung*: dilution factor).

**Figure 8. Comparison of biglycan core protein sequences** (for further details see Fig. 3 of Dreher (1990)).

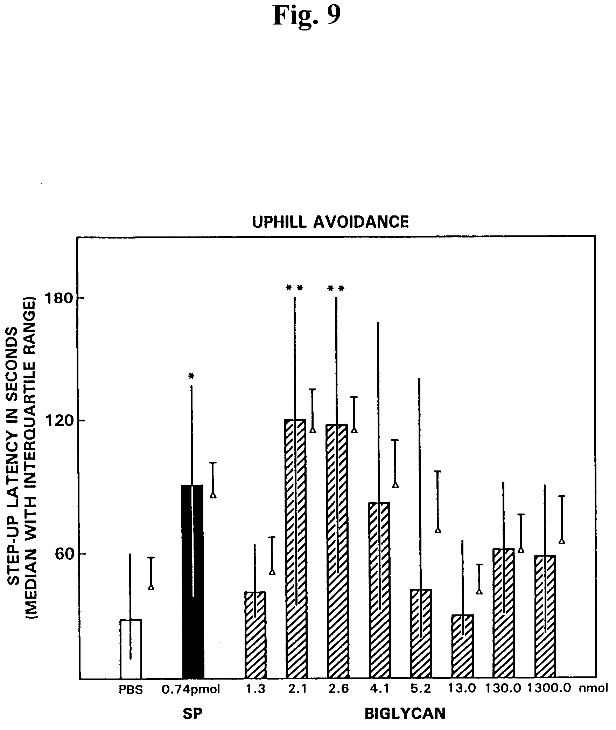

**Figure 9. Effects of 0.74 pmol substance P (SP) and different doses of proteoglycan biglycan on uphill avoidance performance.** During the training trial, the rats received a tail-shock contingent on the uphill response. Retention is expressed as median (with interquartile range) latency to step-up measured 24 hr after the training trial ($\triangle$ to the right of each column indicate respective mean + SEM). The treatment consisted of post-trial injection of 0.74 pmol SP, eight different doses of biglycan ranging from 1.3 to 1300.0 nmol, or vehicle (PBS). The injections were given unilaterally into the NBM area. **, $p < 0.01$; *, $p < 0.05$ versus PBS-vehicle controls; Mann-Whitney $U$ test: SP-group, one-tailed; biglycan-groups, two-tailed).

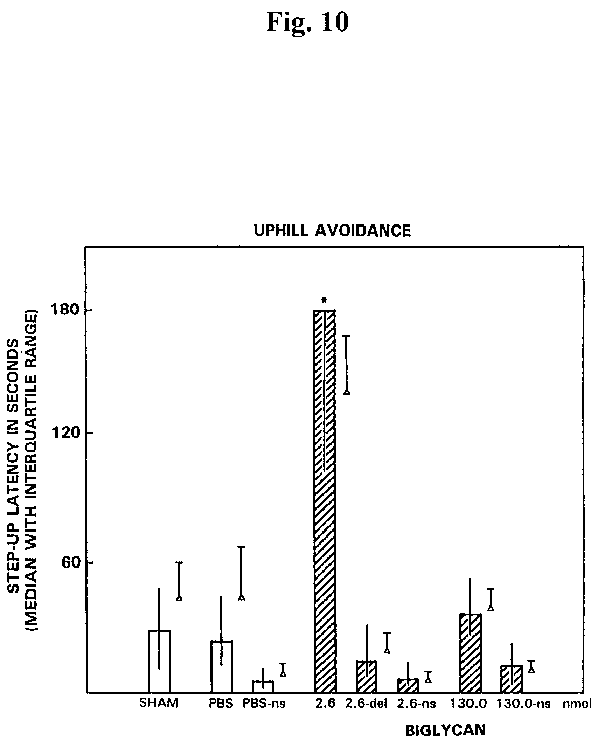

**Figure 10. Effects of proteoglycan biglycan on uphill avoidance performance.** During the training trial, the rats received a tail-shock contingent on the uphill response. Retention is expressed as median (with interquartile range) latency to step-up measured 24 hr after the training trial ($\triangle$ to the right of each column indicate respective mean + SEM). The treatment consisted of post-trial injection of 2.6 nmol or 130.0 nmol of biglycan. Controls included sham-operated (SHAM), vehicle-injected (PBS), 5-hour delay (biglycan 2.6-del) and no shock (-ns) groups. Injections were given unilaterally into the NBM area. *, $p < 0.05$ versus PBS, biglycan 2.6-del and biglycan 2.6-ns groups; Mann-Whitney $U$ test (one-tailed).

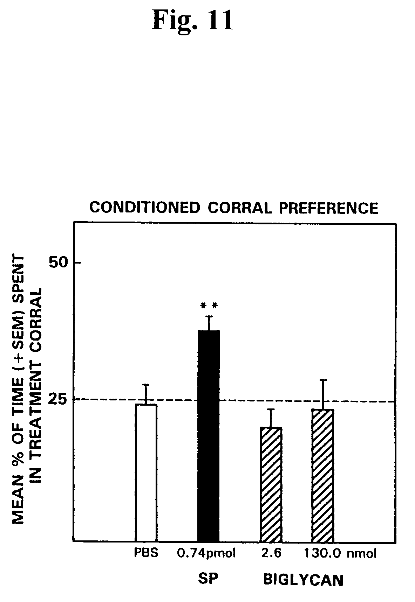

**Figure 11. Mean % of time (+ SEM) spent in the treatment corral during the 15-min observation period on the day of testing in the open Corral.** A single conditioning trial was performed in the Corral apparatus. The rats were injected with SP (0.74 pmol), biglycan (2.6 or 130.0 nmol) or vehicle (PBS) into the NBM region and placed into one of four restricted quadrants of the open field (closed Corral). During test for conditioned place preference in the open Corral the rats could navigate the whole open field. Mann-Whitney $U$ test performed on raw data was used to test for between-group differences (SP-group, one-tailed; biglycan-groups, two-tailed). **, $p < 0.01$ versus PBS-vehicle controls.

**SEQ ID NQ:1. Biglycan cDNA sequence** (for further details see Fig. 1 of Dreher (1990)).

Table 1

| Neurotrophic effect of MCM | | |
|---|---|---|
| Culture Medium | Number of cells (total number/well ± SD) | |
| | 18 h | day 5 |
| N2 | 9032 ± 464 (n = 6) | 795 ± 189 (n = 6) |
| MCM* | 9338 ± 53 (n = 4) | 9836 ± 106 (n = 4) |

*40,7 $\mu$g protein/well that correspond to a saturating amount of neurotrophic activity.

Neocortical neurons were cultured as described in Materials and Methods. Cells were stained with toluidine blue (18 h) or anti-MAP2 monoclonal antibodies (day 5) and all cells (18 h) or MAP2-positive neurons bearing at least one neurite longer than two cell diameters (day 5) were counted. Less than 1% of non-neuronal cells were detected in the cultures at day 5. n: number of wells.

Table 2

| Comparison of purification steps | | | | | |
|---|---|---|---|---|---|
| step | total protein (μg) | ng protein/50% survival | specific activity (TU/mg protein) | purification factor (fold) | yield (%) |
| MCM | 537167 | 2032 | 492 | 1 | 100 |
| Q-Sepharose | 5867 | 125 | 8000 | 16,2 | 17,7 |
| MonoQ | 2200 | 156 | 6410 | 13 | 5,3 |
| Superose 6 | 450 | 108 | 9260 | 19 | 1,6 |

At each stage the activity was assayed as described in Materials and Methods. One TU is defined as the amount of activity which supports half maximal neuronal survival in our bioassay. Maximal survival is observed in response to saturating concentrations of MCM. Protein was determined according to Bradford (1976) with immunoglobuline as standard.

Table 3

| Survival of neocortical neurons in the presence of purified biglycan from MCM after 5 days in culture | |
|---|---|
| Addition | total number of surviving neurons per well ($\pm$ SD) |
| N2 | 795 $\pm$ 189 (n = 6) |
| biglycan, 190 ng/well[*] | 9137 $\pm$ 489 (n = 4) |
| biglycan, 95 ng/well[**] | 6731 $\pm$ 329 (n = 2) |

[*]8946 $\pm$ 424 (n = 2) and
[**]9306 $\pm$ 276 (n = 2) cells were counted per well at 18 h after plating. For cell number counted in N2 confer Table 1; n: number of wells.

**Table 4.** Duration (t) and frequency (f) of behavioral events during the conditioning trial in the closed Corral. The rats were injected with either biglycan (BG 2.6 or BG 130.0 nmol), SP (0.74 pmol) or vehicle (PBS) and placed into one of four restricted quadrants of the open field (closed Corral) for a 15-min period. Values are means ($\pm$ SEM). * $p < 0.05$; Mann-Whitney $U$ test (two-tailed).

| Behavior | | PBS | Treatment SP 0.74 pmol | BG 2.6 nmol | BG 130.0 |
|---|---|---|---|---|---|
| Locomotion | (t) | 121.3 ±11.9 | 169.2 ±28.9 | 139.1 ±9.5 | 99.5 ±11.5 |
| | (f) | 50.9 ±6.0 | 54.0 ±8.6 | 71.7* ±3.0 | 64.9 ±6.9 |
| Rearing | (t) | 206.4 ±27.6 | 138.8 ±21.3 | 143.0 ±14.6 | 146.7 ±18.3 |
| | (f) | 49.2 ±6.3 | 36.1 ±6.1 | 60.1 ±5.5 | 57.0 ±5.6 |
| Grooming | (t) | 117.9 ±17.6 | 89.3 ±22.6 | 65.5 ±13.5 | 78.8 ±15.3 |
| | (f) | 8.8 ±1.1 | 6.6 ±0.9 | 9.0 ±1.4 | 8.7 ±1.5 |

**Table 5.** Mean (± SEM) time in seconds spent in the treatment corral during test for conditioned place preference for rats injected with SP (0.74 pmol), biglycan (BG 2.6 or BG 130.0 nmol), or vehicle. The observation period of 15 min was divided into three time blocks of 5 min. TC, treatment corral. **, $p < 0.01$, *, $p < 0.05$; Mann-Whitney $U$ test (one-tailed).

| | Time spent in TC | | |
|---|---|---|---|
| Treatment | min 0-5 | min 6-10 | min 11-15 |
| PBS | 77.9 ±8.2 | 69.5 ±8.8 | 64.4 ±11.3 |
| SP 0.74 pmol | 92.7 ±9.9 | 100.4** ±10.7 | 127.8* ±22.6 |
| BG 2.6 nmol | 54.4 ±6.3 | 63.7 ±12.7 | 51.2 ±14.0 |
| BG 130.0 nmol | 67.3 ±8.5 | 73.7 ±21.1 | 73.2 ±20.6 |

### References

Ansorge W (1985) Fast and sensitive detection of protein and DNA bands by treatment with potassium permanganate. J Biochem Biophys Methods 11:13-20

Asundi VK, Dreher KL (1992) Molecular characterization of vascular smooth muscle decorin: deduced protein structure and regulation of gene expression. Eur J Cell Biol 59:314-321

Barde Y-A, Edgar D, Thoenen H (1982) Purification of a new neurotrophic factor from mammalian brain. EMBO J 1:549-553

Bianco P, Fisher LW, Young MF, Termine JD, Gehron Robey P (1990) Expression and localization of the two small proteoglycans biglycan and decorin in developing human skeletal and non-skeletal tissues. J Histochem Cytochem 38:1549-1563

Bottenstein JE, Sato GH (1980) Growth of rat neuroblastoma cell line in serum-free supplemented medium. Proc Natl Acad Sci U.S.A. 76:514-517

Bradford MM (1976) A rapid and sensitive method for quantitation of microgram quantities of protein utilizing the principle of protein-dye binding. Anal Biochem 72:248-254

Dreher KL, Asundi VK, Matzura D, Cowan K (1990) Vasular smooth muscle biglycan represents a highly conserved proteoglycan within the arterial wall. Eur J Cell Biol 53:296-304

Fisher LW, Hawkins GR, Tuross N, Termine JD (1987) Purification and partial characterization of small proteoglycans I and II, bone sialoproteins I and II, and osteonectin from the mineral compartment of developing human bone. J Biol Chem 262:9702-9708

Fisher LW, Heegaard A-M, Vetter U, Vogel W, Just W, Termine JD, Young MF (1991) Human biglycan gene. Putative promotor, intron-exon junctions, and chromosomal localization. J Biol Chem 266:14371-14377

Fisher LW, Termine JD, Young MF (1989) Deduced protein sequence of bone small proteoglycan I (biglycan) shows homology with proteoglycan II (decorin) and several non-connective tissue proteins in a variety of species. J Biol Chem 264:4571-4576

Hashimoto C, Hudson KL, Anderson KV (1988) The toll gene of Drosophila, required for dorsal-ventral embryonic polarity, appears to encode a transmembrane protein. Cell 52:269-279

Hausser H, Ober B, Quentin-Hoffmann E, Schmidt B, Kresse H (1992) Endocytosis of different members of the small chondroitin/dermatan sulfate proteoglycan family. J Biol Chem 267:11559-11564

Kyhse-Andersen J (1984) Electroblotting of multiple gels: a simple apparatus without buffer tank for rapid transfer of proteins from polyacrylamide to nitrocellulose. J Biochem Biophys Methods 10:203-209

Laemmli UK (1970) Cleavage of structural proteins during assembly of the head of bacteriophage T4. Nature (London) 227:680-685

Lochter A, Vaughan L, Kaplony A, Prochiantz A, Schachner M (1991) J1/tenascin in sustrate-bound and soluble form displays contrary effects on neurite outgrowth. J Cell Biol 113:1159-1171

Margolis RK, Margolis RU (1993) Nervous tissue proteoglycans. Experientia 49:429-446

Matthiessen HP, Guder A, Müller HW (1991a) Laminin proteoglycan complexes with survival activity for cultured neurons from rat neocortex. In: Synapse - transmission - modulation: Proceedings of the 19th Göttingen Neurobiology Conference. (Elsner N, Penzlin H eds.), p 490. Stuttgart; New York: Thieme.

Matthiessen HP, Schmalenbach C, Müller HW (1991b) Identification of meningeal cell released neurite promoting activities for embryonic hippocampal neurons. J Neurochem 56:759-768

Müller HW, Beckh S , Seifert W (1984) Neurotrophic factor for central neurons. Proc Natl Acad Sci USA 81:1248-1252

Müller HW, Matthiessen HP, Schmalenbach C, Schroeder WO (1991) Glial support of CNS neuronal survival, neurite growth and regeneration. Restor Neurol Neurosci 2:229-232

Patthy L (1987) Detecting homology of distantly related proteins with consensus sequences. J Mol Biol 198:567-577

Reinke R, Krantz DE, Yen D, Zipursky SL (1988) Chaoptin, a cell surface glycoprotein required for Drosophila photoreceptor cell morphogenesis, contains a repeat motif found in yeast and human. Cell 52.291-301

Rosenberg LC, Choi HU, Tang L-H, Johnson TL, Pal S, Webber C, Reiner A, Poole AR (1985) Isolation of dermatan sulfate proteoglycans from mature bovine articular cartilages. J Biol Chem 260:6304-6313

Ruoslahti E (1989) Proteoglycans in cell regulation. J Biol Chem 264:13369-13372

Ruoslahti E, Yamaguchi Y(1991) Proteoglycans as modulators of growth factor activities. Cell 64:867-869

Schulz M, Raju T, Ralston g, Bennet MR (1990) A retinal ganglion cell neurotrophic factor purified from the superior colliculus. J Neurochem 55:832-841

Stöcker G, Meyer HE, Wagener C, Greiling H (1990) Purification and N-terminal amino acid sequence of a chondroitin sulphate/dermatan sulphate proteoglycan from intima/media preparations of human aorta. Biochem J 274:415-420

Carey RJ (1987) Post-trial hormonal treatment effects: memory modulation or perceptual distortion? J Neurosci Methods 22:27-30.

Carr GD, Fibiger HC, Phillips AG (1989) Conditioned place preference as a measure of drug reward. In: The neuropharmacological basis of reward (Liebman JM, Cooper SJ, eds), pp 264-319. Oxford: Clarendon Press.

Fisher LW, Termine JD, Deijter SW, Whitson WW, Yanagishita M, Kimura JH, Hascall V, Kleinman HK, Hassell JR, Nilson B (1983) Proteoglycans of developing bone. J Biol Chem, 258:6588-6594.

Gerhardt P, Hasenöhrl RU, Huston JP (1992) Enhanced learning produced by injection of neurokinin substance P into the region of the nucleus basalis magnocellularis: mediation by the N-terminal sequence. Exp Neurol 118:302-308.

Gold PE (1986) The use of avoidance training in studies of modulation of memory storage. Behav Neural Biol 46:87-98.

Hagan JJ, Morris RGM (1988) The cholinergic hypothesis of memory: a review of animal experiments. In: Handbook of psychopharmacology, vol. 20 (Iversen LL, Iversen SD, Snyder SH, eds), pp. 237-323. New York: Plenum Press.

Hasenöhrl RU, Gerhardt P, Huston JP (1992) Positively reinforcing effects of the neurokinin substance P in the basal forebrain: mediation by its C-terminal sequence. Exp Neurol 115:282-291.

Hasenöhrl RU, Oitzl M-S, Huston JP (1989) Conditioned place preference in the corral: a procedure for measuring reinforcing properties of drugs. J Neurosci Methods 30:141-146.

Heimer L, Alheid GF (1991) Piecing together the puzzle of basal forebrain anatomy. In: The basal forebrain: anatomy to function (Napier TC, Kalivas PW, Hanin I, eds), pp. 1-42. New York: Plenum Press.

Van der Kooy D (1987) Place conditioning: a simple and effective method for assessing the motivational properties of drugs. In: Methods of assessing the reinforcing properties of abused drugs (Bozarth MA, ed), pp. 229-240. New York: Springer-Verlag.

Holzhäuer-Oitzl M-S, Hasenöhrl RU, Huston JP (1988) Reinforcing properties of substance P in the region of the nucleus basalis magnocellularis in rats. Neuropharmacology 27:749-756.

Huston JP, Hasenöhrl RU, Boix F, Gerhardt P, Schwarting RKW (1993) Sequence-specific effects of neurokinin substance P on memory, reinforcement, and brain dopamine activity. Psychopharmacology 112:147-162.

Kafetzopoulos E, Holzhäuer M-S, Huston JP (1986) Substance P injected into the region of the nucleus basalis magnocellularis facilitates performance of an inhibitory avoidance task. Psychopharmacology 90:281-283.

Koob GF (1987) Neuropeptides and memory. In: Handbook of psychopharmacology, vol. 19 (Iversen LL, Iversen SD, Snyder SH, eds), pp. 531-573. New York: Plenum Press.

Martinez JL, Jensen RA, Messing RB, Rigter H, McGaugh JL (1981) Endogenous peptides and learning and memory processes. New York: Academic Press.

Martinez JL, Jensen RA, McGaugh JL (1983) Facilitation of memory consolidation. In: The physiological basis of memory (Deutsch JA, ed), pp. 49-70. New York: Academic Press.

McGaugh JL (1989) Involvement of hormonal and neuromodulatory systems in the regulation of memory storage. Annu Rev Neurosci 12:255-287.

Nagel JA, Huston JP (1988) Enhanced inhibitory avoidance learning produced by post-trial injections of substance P into the basal forebrain. Behav Neural Biol 49:374-385.

Paxinos G, Watson C (1986) The rat brain in stereotaxic coordinates, 2nd ed. New York: Academic Press.

Stäubli U, Huston JP (1979) Up-hill avoidance: a new passive-avoidance task. Physiol Behav 22:775-776.

Swerdlow NR, Gilbert D, Koob GF (1989) Conditioned drug effects on spatial preference. In: Neuromethods, Vol. 13, Psychopharmacology (Boulton AA, Baker GB, Greenshaw AJ eds), pp. 399-446. Clifton: Humana Press.

## Example 1

### Purification of a meningeal cell-derived chondroitin sulphate proteoglycan with neurotrophic activity for brain neurons and its identification as biglycan

### Materials and Methods

### Meningeal cell conditioned medium (MCM)

Meningeal fibroblasts (immunocytochemically $\geq$ 98% fibronectin positive/glial fibrillary acidic protein negative cells) from embryonic rats were prepared as previously described (Matthiessen et al., 1991b). Briefly, the leptomeninges were carefully removed from hemispheres of embryonic day 18 (E 18) Wistar rats and trypsinized with 0.05% trypsin/0.02% EDTA (Boehringer Mannheim, Mannheim, Germany) for 45 min at 37°C. After trituration with cannulas (diameter 0.7 mm and 0.4 mm) the cell suspension obtained from three embryos was seeded in a 75 cm$^2$ flask (Greiner GmbH, Frickenhausen, Germany) in 10% FCS/DMEM (Gibco/BRL, Eggenstein, Germany) and grown to confluency within 5 to 7 days at 37°C in a humidified 10% $CO_2$/90% air atmosphere. The cells were subcultured twice and expanded to a final monolayer area of approximately 10.5 m$^2$ within 4 weeks. For the final passage from 1.2 m$^2$ to 10.5 m$^2$ culture area, 3 x 106 cells/175 cm$^2$ flask (Greiner) turned out to be an optimal plating number. Higher initial cell densities often resulted in serious cell detachment after change to serum-free hormone-supplemented DMEM [(H-DMEM) containing 5 $\mu$g/ml bovine insulin, 100 $\mu$g/ml human transferrin, 20 nM progesterone, 30 nM sodium selenite, 100 $\mu$M putrescine, and 3.9 mM glutamine (all from Sigma, Heidelberg, Germany)]. The confluent monolayer was washed three times with PBS and incubated with H-DMEM (1.14 ml/10 cm$^2$ culture area). After 24 hrs, the medium was discarded and replaced by fresh H-DMEM medium. Every 3-4 days, the serum-free MCM was collected, filtered (0.22 $\mu$m Milli-Fil GS, Millipore, Bedford, MA, U.S.A.) and stored at -70°C. Fresh H-DMEM was provided up to five times.

**Neuronal cell culture and bioassay of survival activity**

**1. Principle.** The survival activity in MCM and chromatographic fractions was quantified by testing the effect of serially diluted samples on the survival of neocortical neurons in a bioassay. Living cells were stained with a chromogenic viability marker and the determined activity was expressed in trophic units (see below).

**2. Preparation of culture dishes.** To provide optimal cell attachment conditions, 96-well tissue culture plastic microtiter plates (Falcon, purchased from Becton Dickinson, Heidelberg, Germany; 0.38 cm$^2$/well) were incubated overnight at 4°C with 0.1 mg/ml poly-D-lysine (Sigma Chemie GmbH, Deisenhofen, Germany) in PBS (Gibco/BRL). The wells were washed three times with PBS and incubated overnight at 4°C with PBS containing 4 $\mu$g/ml mouse EHS laminin (Gibco/BRL) and finally washed again with PBS. The wells were filled with 100 $\mu$l N2 (Bottenstein and Sato, 1980) [4:1 mixture of DMEM/Hams F-12 (Gibco) containing the supplements listed above for H-DMEM], and supplied with either MCM or aliquots of chromatographic fractions dialysed against DMEM (Gibco/BRL) and sterilized by passage through a 0.22 $\mu$m filter (Millex GV4, Millipore). MCM as well as fractions were serially diluted (1:2) and equilibrated in humidified 10% CO$_2$/90% air at 37°C.

**3. Quantification of neuronal viability.** Neocortical neurons were isolated from embryonic day 15 (E15) Wistar rats according to the protocol of Müller et al. (1984). Briefly, after dissection the tissue was incubated in 0.05% trypsin/0.02% EDTA (10 min, 37°C), rinsed with 10% FCS/DMEM to block protease activity, washed two times with DMEM, resuspended in N2, triturated with fire-polished Pasteur pipettes and passed through 30 $\mu$m nylon gauze. Viable cells (determined by fluorescein diacetate stain) were seeded into the laminin-precoated 96-well microtiter plates at a density of approx. 4 x 104 cells/cm$^2$ in 100 $\mu$l N2 per well. The cells were maintained in a total volume of 200 $\mu$l up to 5 days without medium change. The cell number per well was calculated from cell counts in microscopic fields comprising 5% of the culture area. Cells were stained with either toluidine blue (18 hrs after plating) or anti-Map2 antibodies (day 5; see below). The cell numbers in MCM and N2 were identical at 18 hrs after plating (~65% plating efficiency). To determine the survival activity of individual column fractions, neurons were routinely stained with 3-(4,5-dimethylthiazol-2-yl)-2,5-diphenyltetrazolium bromide (MTT) (Sigma, Deisenhofen, Germany) using a final concentration of 150 $\mu$g/ml for 60 min at 37°C in 10% CO$_2$/90% air. The blue formazan precipitate formed by viable cells was solubilized by replacing 100 $\mu$l medium by 100 $\mu$l acidic isopropanol [0.6% (vol/vol) HCl in isopropanol] per well with subsequent sonication (15 sec; SonoRex super RK 255, Fa. Bandelin, Berlin, Germany). The absorbance at 550 nm (measured in multiplate reader MR 500; Dynatech, Deukendorf, Germany) was proportional to the amount of viable cells as determined by counting of sibling cultures. During the course of purification one trophic unit (TU) was defined as that amount of activity supporting half maximal neuronal survival. TUs of individual fractions were determined by plotting the dilution factor versus the corresponding absorption at 550 nm. On each 96-well microtiter plate serially diluted MCM of the respective preparation was used as reference for maximal neuronal survival and wells with N2 medium with and without neurons served to estimate the background.

**Centrifugation assay for neuron-substratum adhesion**

U-well multititer plates (96 wells, Greiner GmbH) were preincubated overnight at 4°C with PBS containing 4 $\mu$g/ml mouse EHS laminin (Gibco/BRL), 5 and 10 $\mu$g/ml biglycan purified from MCM or PBS, respectively. After washing three times with 10 mg bovine serum albumin/ml PBS (BSA, Sigma) the plates were blocked by incubation with the BSA solution for 4h at 37°C. Neocortical neurons freshly prepared from E15 rats were plated at a density of 5 x 104 cells per well. Immediately thereafter the plates were centrifuged at 250 g in a swing-out rotor for 2 min and the diameter of the cell pellets was measured using an inverted microscope.

**Histochemical and immunochemical staining of cultured neurons**

Cells were fixed with 4% (wt/vol) paraformaldehyde (Merck, Darmstadt, Germany) in PBS for 15 min at room temperature (RT). For histochemical staining (Lochter et al., 1991), cells were washed twice with PBS and stained for 15 min with 0.5% (wt/vol) toluidine blue O (Boehringer Mannheim) in 2.5% (wt/vol) Na$_2$CO$_3$, washed twice with distilled water, air dried and covered in DPX (Fluka, Neu-Ulm, Germany). Immunocytochemistry using monoclonal mouse anti-microtubule-associated protein 2 (Map2) antibodies (Sigma) was carried out according the protocol of Stichel and Müller (1992). Fixed cells were washed with PBS, preincubated for 30 min at room temperature in PBS containing 10% FCS/2% BSA/0.6% Triton X-100 and subsequently incubated for 18 to 24 hrs at 4°C with anti-MAP2 antibody (1:500) in PBS/0.6% Triton X-100. To detect bound primary antibodies the Vectastain-Elite-ABC-Kit (Camon, Wiesbaden, Germany) was used according to a modification of the manufacturers protocol. In brief, cells were washed three times with PBS, incubated for 45 min at RT with biotinylated horse anti-mouse IgG (1:200 in PBS/0.6% Triton X-100), and, following three washes with PBS, incubated for 30 min at RT with ABC-complex 1:100 in PBS/0.6% Triton X-100. Subsequently the cells were washed three times with 50 mM Tris/HCl pH 7.5 (Tris), incubated with 0.5 mg/ml diaminobenzidine, 0.015% (vol/vol) $H_2O_2$ in Tris for 2 min at RT, washed twice with distilled water and either air dried or covered in Aquatex (Merck). As a control the primary antibody was omitted in otherwise identically treated cultures. Under these conditions, no specific staining was observed. In some experiments the nuclei were counterstained with Mayers haemalaun to determine the proportion of non-neuronal cells in the neocortical cultures (less than 1% at day 5).

**Purification procedure**

For a typical preparation, 3 to 5 l of MCM (stored at -70°C) were carefully thawed in a water bath (37°C) under continous shaking and then immediately transferred to 4°C and passed through a preparative Q-Sepharose column [bed volume 300 ml (Pharmacia/LKB, Freiburg, Germany)] with a flow rate of approx. 15 cm/h. The bound material was eluted with a two-step salt gradient (1 l of 0.6 M NaCl and 1 l of 1.2 M NaCl in 50 mM Tris/HCl pH 7.4, respectively) and collected in 10 ml fractions.. Fractions containing the neurotrophic activity were eluted with 1.2 M NaCl ("Q-Sepharose-Pool") and dialyzed against MilliQ water. For all dialysis steps bags with a mol. wt. cut-off of 2000 (Spectra/Por 6 dialysis tubing, Serva, Heidelberg, Germany) were used. After lyophilization the resulting protein pellet was resuspended in NaAc buffer A (50 mM sodium acetate pH 6.0, 70 mM NaCl, 8 M urea), stored overnight at 4°C, filtered (0.22 $\mu$m) and rechromatographed on a MonoQ (5/5) column (Pharmacia/LKB) in the presence of 8 M urea (ultra pure; BRL, Life Technologies Inc., Gaithersberg, MD 20877, U.S.A). The bound material was eluted at a flow rate of 1 ml/min with a linear gradient of sodium chloride ranging from 0.07 M to 1.4 M NaCl in 50 mM NaAc pH 6.0/8 M urea. Active fractions (1 ml each) were exclusively eluted at salt concentrations above 0.8 M NaCl, combined ("MonoQ-Pool"), and dialyzed against MilliQ water. The lyophilized MonoQ-Pool was resuspended in 500 $\mu$l 50 mM Tris/HCl pH 7.4 containing 4 M guanidinium hydrochloride (ultra pure; BRL, Life Technologies Inc.) and, after an overnight incubation at 4°C, loaded on a Superose 6 (30/10) column (Pharmacia/LKB) equilibrated with the same buffer. The sample was chromatographed with a flow rate of 0.2 ml/min and 0.6 ml fractions were collected. The values of V0 (elution volume of dextran blue) and $V_c$ used to calculate the $K_{av}$ of the neurotrophic activity were 7.2 ml and 24.0 ml, respectively.

**Treatment with glycosaminoglycan:lyases**

Equal amounts of the Q-Sepharose-Pool were incubated with (i) chondroitinase buffer (CB, 50 mM Tris/HCl pH 7.3, 50 mM NaAc, 0.01% BSA) alone, (ii) CB with 6 mU protease-free chondroitinase ABC/mg protein (Seikagaku Kogyo Co. Ltd., Tokyo, Japan), (iii) CB with 6 mU protease-free chondroitinase AC/mg protein (Seikagaku), (iv) heparitinase buffer (HB, 20 mM CaAc in CB) alone, and, (v) HB with 0.2 mU protease-free heparitinase/mg protein (Seikagaku), for 4 h at 37°C (i,ii,iii) or 4 h at 42°C (iv,v), respectively. To remove the glycosaminoglycan (GAG) lyases prior to the bioassay, each sample was rechromatographed on MonoQ (5/5) with a linear gradient of sodium chloride ranging from 0.1 to 2.0 M NaCl in 50 mM Tris/HCl pH 7.4. One ml fractions were collected and tested for survival activity.

**Digestion of biglycan with trypsin**

Equal amounts of lyophilized biglycan (each corresponding to about 160 TU/ml) were resuspended in 50 $\mu$l DMEM and subjected to one of the following treatments: (i) storage at 37°C for 4 h; addition of either (ii) 200 BAEE Units/ml trypsin (Sigma; from a stock of 1.000 U/ml DMEM), (iii) 2.000 BAEE-inhibitory Units/ml soybean trypsin inhibitor (Sigma; stock 10.000 U/ml DMEM) or, (iv) trypsin plus inhibitor, and subsequent incubation at 37°C for 4 h. The sample treated with trypsin alone was then supplied with 2.000 U/ml soybean trypsin inhibitor. At the end of each treatment the samples were diluted with DMEM to 1 ml, filtered and tested for neurotrophic activity.

**Analytical procedures**

Protein was determined according to Bradford (1976) using bovine gamma globuline (Bio-Rad Laboratories, München, Germany) as a standard. SDS-PAGE was performed as described by Laemmli (1970). All samples were denatured in SDS-sample buffer containing 100 mM dithiotreitol (Boehringer Mannheim) for 3 min at 95°C and were immediately cooled down on ice. Gradient slab gels (3-10% wt/vol acrylamide/bisacrylamide) were electrophoresed overnight (15 to 16 h) at RT with 100 V in a ProteanII apparatus (BioRad) and were subsequently stained with silver (Ansorge, 1985).

For NH2-terminal sequencing and analysis of amino acid composition, 100 $\mu$g of active material eluted from the Superose 6 column was digested with 10 mU/$\mu$g protease free chondroitinase ABC (Seikagaku) for 4 h at 37°C in CB buffer (see above). After electrophoresis on a 12% SDS-polyacrylamide gel proteins were transferred to Immobilon-P membrane (Millipore, Bedford, MA) by semi-dry blotting (Kyhse-Andersen, 1984) in a discontinuous buffer system (anode buffer I: 0.3 M Tris/20% methanol; anode buffer II: 0.025 M Tris/20% methanol; cathode buffer: 0.04 M epsilon-aminocaprionic acid/20% methanol). After equilibration of the gel in cathode buffer for 15 min at RT the transfer was accomplished using a NovaBlot SemiDry apparatus (Pharmacia/LKB) with 0.8 mA/cm$^2$ for 2 h. To locate the core protein prior to sequencing the membrane was stained for 15 min with 0.1% Coomassie R 250 (Serva) in 50% methanol/10% acetic acid followed by destaining in 45% methanol/7% acetic acid. Sequence analysis was performed with a model 476 pulse liquide protein sequenator from Applied Biosystems (Weiterstadt, Germany), using the fast blot protocol.

**Results**

**Meningeal cell conditioned medium (MCM) supports survival of cultured embryonic rat neocortical neurons**

Primary neurons from rat neocortex (E15) were cultured with either MCM or N2 medium on a PDL/LN-substrate. 18 h after plating the number of attached cells was almost identical under both medium conditions (Table 1). Neurons cultured in N2 started to die within two days and less than 10% of these cells survived for 5 days after plating. On the other hand, the entire population of neocortical neurons in MCM was still viable on day 5. MCM enhanced neuronal survival in a dose-dependent manner (Fig. 1) and half maximal neuronal survival was achieved at a protein concentration of approx. 10 $\mu$g/ml.

**Purification procedure**

The neurotrophic agent present in MCM could be purified to homogeneity in three steps: (i) initial Q-Sepharose chromatography; (ii) rechromatography on MonoQ in the presence of 8 M urea; and (iii) final gel filtration on Superose 6 in the presence of 4 M guanidinium hydrochloride.

**Step I**

In the experiment shown in Figure 2, 3.3 l MCM were passed over a preparative Q-Sepharose anion-exchange column. The resulting break-through displayed no residual biological activity. The bound material was eluted with a two-step salt gradient (0.6 M and 1.2 M NaCl, respectively) and each fraction was assayed for protein concentration and neurotrophic activity. Neurotrophic activity was eluted with 1.2 M sodium chloride (Fig. 2, Table 2) and active fractions (127 to 138) were pooled ("Q-Sepharose-Pool").

**Step II**

The Q-Sepharose-Pool was rechromatographed on MonoQ in the presence of 8 M urea at pH 6.0. The neurotrophic activity eluted in a single protein peak at ~0.9 M NaCl (Fig. 3), indicating that even under strong dissociative conditions the neurotrophic activity remained associated with a highly negatively charged molecule. SDS-PAGE revealed a marked increase in the stage of purification (Fig. 5).

**Step III**

The final purification step was accomplished by gel filtration on a Superose 6 column performed in 4 M guanidinium hydrochloride. This chaotropic agent interferes with non-covalent intermolecular bonds. The neurotrophic activity was eluted at a $K_{av}$ of 0.21 corresponding to Mr >669 kDa (Fig. 4). Silver staining of SDS-gels at this stage of purification revealed a single protein band migrating as a smear in the molecular weight range of approx. 220-340 kDa (Fig. 5). Approx. 150 $\mu$g protein was obtained per litre MCM with a final recovery of 1.6% of the initial activity (Table 2).

When the gel filtration was carried out under non-denaturating conditions the neurotrophic activity was eluted in the void volume, probably due to aggregate formation by homo- or heterophilic molecular interactions.

## Characterization of the purified molecule

The purification procedure to isolate the neurotrophic activity pointed to a highly negatively charged molecule with a large hydrodynamic diameter in the presence of a chaotropic agent. SDS-gel electrophoresis of the active material obtained after gel filtration revealed one single broad protein band in the range of 220 to 340 kDa. These biochemical properties are typical for proteoglycans. Digestion of Q-Sepharose-Pool with different glycosaminoglycan lyases (for experimental details see Materials and Methods) revealed, that treatment with chondroitinase ABC and AC dramatically reduced the neurotrophic activity to 10% of control, whereas heparitinase treatment had no significant effect on neuronal survival As predicted from these experiments the purified molecule was, indeed, sensitive to chondroitinase but not to heparitinase digestion: The broad protein smear on SDS-polyacrylamide gels was converted into a sharp core protein band of approximately ~50 kDa by digestion with both chondroitinase ABC and AC but remained unaffected by heparitinase treatment. Since treatment with chondroitinase ABC and AC (the latter enzyme does not degrade dermatan sulfate which contains L-iduronic acid) yielded a core protein of equal size, the neurotrophic proteoglycan appears to be predominantly if not exclusively substituted with chondroitin sulfate chains.

Amino acid sequencing of the first twenty-nine N-terminal residues of the neurotrophic CSPG revealed identity to the respective amino acid sequence deduced from rat smooth muscle cell biglycan cDNA (Dreher et al., 1990). The serine residues in position 5 and 11 were found to be modified and they behaved very similar to the glycosylated serines previously identified in another chondroitin/dermatan sulfate proteoglycan (Stöcker et al., 1990). Furthermore, analysis of the total amino acid composition indicated a high proportion of leucine residues (17.6%, data not shown) corresponding well with 15.2% of leucine residues predicted from the rat biglycan cDNA sequence. The gel electrophoretic mobility of the purified CSPG before and after glycosaminoglycan lyase treatments resembled the behavior of "authetic" biglycan from nonneural tissue (e.g. Hausser et al., 1992).

## Biological activity of purified biglycan

Figure 6 shows the effect of purified neurotrophic CSPG/biglycan on survival of cultured embryonic neocortical neurons. The neurotrophic activity of biglycan is dosage-dependent (Fig. 6 E, Table 3). Half-maximal neuronal survival is supported by concentrations of about 100 ng biglycan/well with saturating protein concentrations at approx. 200 ng/well. Thus, the effective concentration resides in the nanomolar range with a half-maximal effect at $10^{-8}$ M. When the neurotrophic activity of purified biglycan of meningeal cells was compared to the activity of biglycan isolated from bovine cartilage both dose-response curves were virtually identical (Fig. 7).

To test whether biglycan may be an adhesive substrate for neocortical neurons, we performed a micro-well centrifugation assay to estimate the strength of cell-substratum interactions (see Materials and Methods). When cells adhere strongly to the substratum of U-shaped wells, the size of pellets formed after centrifugation is large in comparison to substrates lacking cell adhesive properties. As a substrate in this assay biglycan did neither show adhesive effects nor interfere with laminin-promoted neuron-substrate adhesion, indicating that the observed survival enhancing effect of biglycan is not due to a modification of cell-substrate adhesion.

## Centrifugation assay for neuron-substratum adhesion

U-well microtiter plates were coated with biglycan (BGN) purified from MCM and/or laminin (LN) at the concentrations indicated. 5 x $10^4$ neurons were plated per well and immediately centrifuged at 250 g for 2 min. The diameter of the pellets formed reflects thte adhesiveness of the respective substratum.

## Discussion

We have purified a neurotrophic proteoglycan from MCM that significantly sustains the survival of cultured rat neocortical neurons. During the course of purification strong denaturing conditions were employed to dissociate potential non-covalently bound molecules. As a result, the biological activity was exclusively eluted at high salt concentrations from MonoQ even in the presence of 8 M urea and was further recovered as a molecule with a large hydrodynamic diameter from Superose 6 in the presence of 4 M guanidinium hydrochloride. Silver stained 3-10% SDS-gels of active Superose 6 fractions revealed a single broad glycoprotein band migrating with an apparent size of ~300 kDa. A 90% reduction in the neurotrophic survival activity was achieved by treatment with chondroitinase ABC and AC but not with heparitinase. Likewise, the electrophoretic mobility of the purified molecule changed after digestion with either chondroitinase ABC or AC from ~300 kDa to a ~50 kDa core protein band thus identifying the purified molecule as a chondroitin sulfate proteoglycan.

The electrophoretic mobility of the purified chondroitin sulfate proteoglycan in SDS-polyacrylamide gels resembled that of the small proteoglycans biglycan and decorin of human bone and bovine cartilage (Fisher et al., 1987; Rosenberg et al., 1985). Although mature biglycan and decorin show differences in their apparent Mr of approximately ~300 kDa and ~100 kDa, respectively (Rosenberg et al., 1985), the core proteins are very similar in size (approximately 45 to 50 kDa; Hausser et al., 1992; Fisher et al., 1987; Rosenberg et al., 1985). Yet the amino-terminal sequences of both proteins are very different (Dreher et al., 1990; Asundi and Dreher, 1992). Partial sequencing of the core protein of the purified neurotrophic chondroitin sulfate proteoglycan from meningeal cells revealed identity with rat biglycan but not with decorin.

Purified rat biglycan is biologically active at nanomolar concentrations (half maximal activity approx. $10^{-8}$ M; see Fig. 6, Table 2). The activity of biglycan is lower than the activities normally found for neurotrophic peptide growth factors like, e.g., NGF or BDNF (half maximal actvity at least $3 \times 10^{-11}$ M ; Barde et al., 1982).

Biglycan is an evolutionary highly conserved molecule of yet unknown biological function that has been located on the cell surface or in the pericellular space of a variety of specialized cell types, including differentiating mesenchymal, endothelial and epithelial cells (Bianco et al., 1990). Since biglycan expression dominates at sites of tissue differentiation and growth plate formation a role in cell regulation has been suggested (Bianco et al., 1990). Furthermore, the biglycan core protein is characterized by eleven leucine-rich repeat motifs which were found in a variety of proteins such as the morphoregulatory proteins toll and chaoptin in Drosophila (Hashimoto et al., 1988; Reinke et al. 1988), and which appear to be used throughout evolution to mediate protein-protein, protein-cell and cell-cell interactions (Patthy, 1987). The neurotrophic activity of biglycan described here shows a cell regulatory function of biglycan and a function of this protein in neural cells (see Example 2).

Addition of TGF-$\beta$ (up to 10 ng/ml) to neocortical cultures neither influenced neuronal survival nor inhibited the neurotrophic activity of biglycan, but, instead, slightly modified the expression of biglycan in cultured astrocytes.

## Example 2

**Facilitation of learning following injection of biglycan into the ventromedial pallidum**

## Materials and Methods

### Animals

The experiment was performed on 205 male Wistar rats, weighing 250-320 g at the beginning of the experiments. They were housed two per cage under a 12:12 hr light:dark cycle, with food and water continuously available. All testing was conducted during the rats' daylight period between 10.00 and 16.00 hr.

### Surgery and recovery

Animals were food-deprived for 24 hr prior to surgery. Under Equithesin anesthesia (3.0-4.0 ml/kg), 193 of the rats were unilaterally implanted (50% right side implantation; 50% left side implantation) with a stainless steel cannula (22 gauge; Behr Labor-Technik, Germany) aimed at 1.5 mm above the injection site. The coordinates for injection were 1.3 posterior to bregma, 2.8 mm lateral to the midline and 7.0 mm below the skull surface (Paxinos and Watson, 1986). The guide cannula contained a stylet which extended 0.1 mm beyond the tip. The cannula was attached to the skull by means of acrylic cement and stainless-steel screws. Twelve of the rats received a sham-operation consisting of anesthesia, insertion of the earbars, and scalp incision. Postoperatively the animals were allowed to recover for at least 1 week before initiation of any behavioral testing. During this interval the rats were handled and weighed daily. At the beginning of the behavioral tests, all animals had recovered to at least 90% of their preoperative body weight.

**Drugs and injection procedure**

The undecapeptide substance P (SP; mol.wt 1347.0) was purchased from Peninsula Labs (USA) and prepared for intracranial injection according to the method described elsewhere (Hasenöhrl et al., 1992). The dose of SP used was 0.74 pmol (1 ng). This dose had repeatedly been found to facilitate avoidance learning following post-trial injection and to serve as a reinforcer in a place preference task upon injection into the NBM area (Huston et al., 1993) and was used as a reference in the present experiments. Biglycan was purified from serum-free rat meningeal cell conditioned medium as described in detail in Example 1. The site of injection was within the ventromedial pallidum and includes the nucleus basalis magnocellularis (NBM) which has been implicated in memory (Hagan and Morris, 1988) and reinforcement processes (Huston et al., 1993 for review). The NBM is the major source of the ascending cholinergic forebrain system (Heimer and Alheid, 1991) and the target for the neurotrophic effects of several growth factors. In each experiment one group of animals was treated with a reference dosage of the neurokinin substance P (SP), which has previously been shown to facilitate memory and to exhibit reinforcing effects upon injection into the NBM region (Huston et al., 1993). For intracranial injection the purified proteoglycan biglycan was diluted in phosphate-buffered saline (PBS; pH 7.6). Control animals received this diluent vehicle. All solutions were blind-coded to eliminate bias. Intracranial injections were made into hand restrained rats in a volume of 0.5 $\mu$l per animal administered over 27 seconds with a 28-gauge injection-cannula (Behr Labor-Technik, Germany) inserted to a depth of 1.5 mm below the tip of the guide cannula (50% right side injection; 50% left side injection). The injection cannula was connected via polyethylene tubing to a 5-$\mu$l Hamilton syringe driven by a microsyringe pump (Harvard Apparatus Compact Infusion Pump). Upon completion of the injection, the injection cannula was left in place for an additional 60 seconds to allow for diffusion of the substances away from the cannula tip and to avoid withdrawal of fluid during removal of the cannula. After withdrawal of the injection cannula, another 0.5 $\mu$l of the solution was ejected to check for possible clogging.

**Apparatus**

Behavioral testing was conducted in sound-protected experimental chambers with dim overhead lighting. Masking noise (68 dB) was provided by a noise generator. The behavior of the animals throughout the experiments was recorded by a video system. The experimental apparatus for one-trial uphill avoidance learning has been described previously (Stäubli and Huston, 1979). Briefly, it consisted of a grey Plexiglas box tilted at a 20° angle with 35 cm high walls. Its 50 x 50 cm floor was covered with wire mesh netting, which allowed an easy foothold for the animals. Electrical shock (1.3 mA for 1 second) was delivered through a bipolar ring electrode attached to the tail of the animal and connected to a shock generator.

Place preference conditioning was conducted by the use of the "Corral method", an improved version of the conditioned place preference (CPP) paradigm. The Corral method as well as its validation with morphine and substance P was described in detail elsewhere (Hasenöhrl et al., 1989; Hasenöhrl et al., 1992). Briefly, the Corral apparatus used was a circular open field (83 cm in diameter) with white walls (45 cm high). Different visual and tactile cues were absent within the open field. Spatial orientation was provided by distinct cues situated in the observation chamber around and above the open field. During treatment, the open field was divided by Plexiglas barriers into 4 quadrants (i.e. "closed corrals") of equal size. An important feature of the Corral method is that unlike in the conventional place preference tasks (Carr et al., 1989; Swerdlow et al., 1989, for review), it is not necessary to establish a choice bias before conditioning. With the use of 4 corrals, a significant place conditioning requires an increase in time added to 25% baseline-level, rather than 50% which is the case in an unbiased two compartment situation (Van der Kooy, 1987). Furthermore, with the Corral method, environmental conditions are easily arranged to prevent a significant baseline bias for a certain quadrant of the open Corral.

**Experiment 1: Effects of post-trial biglycan injection into the NBM area on uphill avoidance performance**

In the first experiment, animals received intracranial injections of either biglycan (BG) in doses of 1.3, 2.1, 2.6, 4.1, 5.2, 13.0, 130.0 and 1300.0 nmol, or the diluent vehicle (PBS). Furthermore, SP was administered in the reference dosage of 0.74 pmol, which had been found to enhance uphill avoidance performance in previous experiments (Kafetzopoulos et al., 1986; Gerhardt et al., 1992). The number of animals per group ranged from 9 to 22. The solutions were injected into the NBM area immediately after the training trial of the uphill avoidance task. The experiment was performed on 3 consecutive days. Each rat had to undergo a habituation trial in the uphill paradigm (day 1), during which it was fitted with the tail-electrode and then placed into the center of the inclined box with its nose facing the base. An "uphill response", that is the negative geotaxic behavior, consisted of turning around towards the top of the inclined plane. This turning response was defined as completed as soon as the position of the animal changed by at least 90° in either direction as defined by a horizontal plane formed by the base of the tail and one of the forepaws. During the training trial (day 2) the rats received a single tail-shock contingent upon the uphill response. After shock administration the rats were removed from the box, disengaged from the tail electrode and were injected intracranially with SP (0.74 pmol), BG (dose range 1.3 to 1300.0 nmol) or PBS. Thereafter, the animals were returned to their home cage. Retention of the uphill response was measured 24 hr after shock administration. The rat was fitted with the tail-electrode, placed on the incline with its nose facing the bottom and allowed up to 180 seconds to step-up.

The Wilcoxon rank sum test was used to compare the step-up latency of each group registered during training and retention trial. The Mann-Whitney $U$ test was performed to test for between group differences in step-up latencies for the retention trial. $p$-values reported are one-tailed for the SP-group and two-tailed for BG-groups. For a subsequent descriptive analysis of the retention latencies, the percentage of rats from each group in the latency categories (short, < 60 s ; medium, 60-120 s; > 120 s) was determined.

### Experiment 2: Test for proactive effects of post-trial biglycan on retention performance and test for reinforcing effects of the proteoglycan

In experiment 2, a test for possible proactive effects of post-trial biglycan on performance during the retention trial was performed; furthermore, the uphill avoidance task was combined with the Corral method to assess possible reinforcing effects of biglycan. Groups receiving two different doses of biglycan (2.6 and 130.0 nmol) or vehicle (PBS) into the NBM area immediately after the training trial were tested on the uphill task as described in experiment 1. Control groups included a sham-operated group (SHAM), a biglycan 2.6 nmol 5-hr delayed injection group (BG 2.6-del), and no shock controls that received 2.6 and 130.0 nmol biglycan or vehicle but no tail-shock (BG 2.6-ns; BG 130.0-ns; PBS-ns). Subsequent to training in the uphill task the animals that received biglycan but without tail-shock were tested in the Corral apparatus along with a group that was injected with 0.74 pmol SP. This dose of SP had previously been found to be positively reinforcing after injection into the NBM (Holzhäuer-Oitzl et al., 1988; Hasenöhrl et al., 1992) and served as a reference in the present experiment. The number of animals per treatment group ranged from 7 to 19.

The effects of biglycan and SP on rats' performance in the uphill avoidance and conditioned place preference task were investigated by combining the two paradigms as follows: Each rat had to undergo a habituation (day 1) and a training trial (day 2) on the uphill paradigm as described in experiment 1. After receiving the tail-shock (with the exception of the no-shock controls which received no tail-shock), the subject was immediately removed from the apparatus and injected with either BG 2.6, BG 130.0, PBS, BG 2.6-ns, BG 130.0-ns, or PBS-ns. The remaining animals (SHAM and BG 2.6-del) were held for approximately 2 minutes. After receiving the injection, the animals were returned immediately to their homecage. Five hours after administration of the shock, the animals in the BG 2.6-del group received an injection of 2.6 nmol biglycan. Animals that had received biglycan, SP or vehicle but without tail-shock were placed into one of the four restricted quadrants of the Corral apparatus for a single 15-min conditioning trial (treatment corral balanced within each group; corral assignment randomly). In order to gauge acute effects of the SP or biglycan treatment on spontaneous behavior, the behavior during the conditioning trial was subjected to a detailed analysis. Duration and frequency of the following behavioral events were registered (see Hasenöhrl et al., 1992 for details): *locomotion* - forward or backward movements using all limbs; *rearing* - standing on the hindlegs with forelegs in the air or against the wall; *grooming* - licking, scratching, biting of the body surface. After the treatment trial in the Corral the animals were returned to their homecage. Retention of the uphill response was measured 24 hr after shock administration as described for experiment 1. Subsequent to the retention trial rats of the no-shock groups had to undergo a test for conditioned place preference in the Corral apparatus. For this purpose, the Plexiglas barriers were removed, giving the rat free access to all parts of the open Corral for 15 min.

The statistical analysis of the step-up latencies registered during training and retention trial on the uphill avoidance task was identical to that described for experiment 1, except that all $p$-values reported are one-tailed values. Between-group differences in duration and frequency of the behavioral events during the 15-min observation period in the closed Corral were evaluated with the Mann-Whitney $U$ test (two-tailed). A reinforcing effect of the drug treatment in the open Corral was defined by the occurrence of place preference behavior, as indicated by an increase in the amount of time spent in the treatment corral on the day of testing. Between-group differences in the amount of time spent in the treatment corral during the whole test period of 15 min as well as during the three time blocks of 5 min (min 0-5, min 6-10, min 11-15) were evaluated with the Mann-Whitney $U$ test (SP-group, one-tailed; biglycan-groups, two-tailed). The frequency of entries into the quadrants during test for conditioned place preference were subjected to descriptive analysis (mean ±SEM).

## Results

### Experiment 1: Effects of post-trial biglycan injection into the NBM area on uphill avoidance performance.

The median step-up latencies for the treatment groups on the training trial fell within the range of 1.2 (1.0-2.0) to 6.9 (4.9-11.0) seconds (median with IQR). Comparisons between training and retention latencies indicate that all groups had learned the task (corresponding $p$'s < 0.05). The effect of SP and the different doses of biglycan on retention performance are depicted in Figure 9. Post-trial injection of 0.74 pmol SP into the NBM facilitated learning of the uphill avoidance task, as reflected by an increase in the latencies to step-up ($p = 0.014$) and by a different distribution of the retention scores compared to PBS-treated animals. The treatment caused a shift to the medium and long latency category in 40% more rats compared to PBS injection. Post-trial injection of biglycan into the NBM area also facilitated performance of the uphill avoidance task and this facilitation appeared to be dose-dependent. Compared to vehicle-injected rats, animals in the BG 2.1 as well as BG 2.6 nmol groups had significantly longer step-up latencies (BG 2.1 nmol, $p = 0.006$; BG 2.6 nmol, $p = 0.003$); fifty-three % more animals from the two groups failed to step-up within 60 seconds. The facilitatory effect on avoidance performance was weaker in rats treated with 4.1 nmol biglycan (BG 4.1 nmol, $p = 0.055$). The higher doses (5.2 to 1300.0 nmol) and the lower dose of biglycan (1.3 nmol) did not influence avoidance performance (corresponding $p$'s between 0.159 and 0.622). Rats treated with 2.1 or 2.6 nmol biglycan did not differ from SP-injected rats (step-up latency: corresponding $p$'s > 0.15).

### Experiment 2: Test for proactive effects of post-trial biglycan on retention performance and test for reinforcing effects of the proteoglycan.

The median step-up latencies for the treatment groups on the training trial ranged from 1.5 (1.2-4.5) to 4.6 (3.1-6.1) seconds (median with IQR). Comparisons between training and retention values indicate that only those groups learned the task which had received a shock after the turning response ($p$'s between 0.014 and 0.023; no-shock groups, $p$'s > 0.05). Comparisons between retention test latencies of the different treatment groups indicated that the BG 2.6 nmol treatment group showed better avoidance as reflected by an increase in the latencies to step-up ($p = 0.038$; Fig. 10) and by a different distribution of the retention scores compared to PBS-vehicle treated animals. The treatment caused a shift to the medium and long latency category in 61% more rats compared to PBS injections. In contrast, the higher dose of biglycan did not influence avoidance performance (BG 130 nmol, $p = 0.112$). When biglycan in a dosage of 2.6 nmol was injected 5 hours after training, it no longer facilitated performance of the task. The latter animals rats did not differ from vehicle-injected controls ($p = 0.385$) and had shorter step-up latencies than rats receiving 2.6 nmol of biglycan immediately after shock administration ($p = 0.010$). This indicates that the facilitative effect of biglycan was not due to long-lasting proactive effects on performance during the retention test session. Sham-operated animals did not differ from vehicle-injected controls ($p = 0.368$) suggesting that insertion of the cannula and/or injection of the vehicle did not affect performance. Step-up latency of rats receiving 2.6 or 130.0 nmol biglycan but no shock did not differ from no shocked controls (BG 2.6-ns, $p = 0.500$; BG 130-ns, $p = 0.185$). Animals in the no-shock groups showed impaired performance in comparison with the respective groups receiving biglycan or vehicle in combination with shock ($p$'s between 0.0002 and 0.008). Thus, the performance enhancing effects of biglycan cannot be attributed solely to nonspecific effects such as increased arousal and/or attention or aversive side-effects.

Table 4 presents duration (t) and frequency (f) of the behavioral events registered during the conditioning trial in the restricted quadrants of the Corral apparatus. The frequency of locomotion was increased for rats that had received an NBM injection of 2.6 nmol BG ($p = 0.042$). Furthermore, rats injected with 2.6 nmol BG spent less time grooming ($p = 0.088$). In contrast, rats treated with 130.0 nmol BG or 0.74 pmol SP did not differ in their behavioral pattern from vehicle-treated rats either in the duration or in the frequency of the behaviors (corresponding $p$'s > 0.100). Figure 11 shows the amount of time spent in the treatment corral during the 15-min test session for rats that had received biglycan (2.6 or 130.0 nmol) or vehicle immediately after the training trial on the uphill task but without tail-shock or SP in the reference dosage of 0.74 pmol. Rats treated with vehicle spent a comparable amount of time in each of the four quadrants of the open corral (about 25% of time in the treatment corral). Rats injected with 0.74 pmol SP spent more time in the treatment corral compared to vehicle controls ($p = 0.008$); about 67% of the rats showed an absolute corral preference in that they spent most time in the treatment corral compared to the other quadrants of the open corral during test. The increase in time spent in the treatment corral for SP-treated rats was most pronounced during the 6- to 10-min ($p = 0.009$) and 11- to 15-min time periods ($p = 0.011$; Tab. 5). In contrast, none of the doses of biglycan influenced rats' preference behavior neither during the whole observation period of 15 min (BG 2.6 nmol, $p = 0.248$; BG 130.0 nmol, $p = 8623$), nor during the three time blocks of 5 min (BG 2.6 nmol: $p$'s between 0.112 and 0.817; BG 130.0 nmol: $p$'s between 0.418 and 1.000). Gross locomotor activity, expressed as the number of entries into the quadrants was not influenced by the SP or biglycan treatment. The mean (± SEM) number of entries into the quadrants ranged from 52.0±8.8 to 69.9±6.3. Furthermore, the treatment did not influence the number of entries into the treatment corral. For each treatment group approximately 25% of the entries were into the treatment corral during test for conditioned place preference (data not shown).

## Discussion

The results of the first experiment provide evidence that post-trial injection of biglycan into the region of the NBM promotes inhibitory avoidance learning in a dose-dependent manner. Injection of 2.1 and 2.6 nmol biglycan significantly improved retention performance, whereas lower or higher doses were ineffective. In addition, the memory-enhancing effect of neurokinin SP revealed in this experiment confirms previous studies (Kafetzopoulos et al., 1986; Nagel and Huston, 1988; Gerhardt et al., 1992). The memory-promoting effects of biglycan were reflected by an inverted $U$-shaped dose-response function which is characteristic for the behavioral pharmacology of peptides (Gold, 1986; Koob, 1987 for review). Similar dose-response effects were previously demonstrated for the effects of SP on memory processing (Huston et al., 1993). This inverted $U$ dose-response relationship obtained for biglycan on avoidance learning may be indicative for a specific and physiological action of the proteoglycan in the NBM.

The results of the second experiment indicate that the facilitatory effects of biglycan on learning are no longer evident when injection is performed 5 hours, rather than immediately, alter the learning trial. This suggests that proactive effects of the treatment on performance during the retention test can not account for the enhanced avoidance response and that biglycan was effective during the period after the learning trial when memory traces are unstable and susceptible to disruptive or facilitative manipulations (Martinez et al., 1983; McGaugh, 1989). Effects on avoidance learning that are dependent on the time of application have also been reported for several other "mnemotrophins", including SP (Nagel and Huston, 1988; Hasenöhrl et al., 1990). It has been suggested that the insertion of the cannula and/or injection of the vehicle into the NBM can have disruptive effects on avoidance performance (Kafetzopoulos et al., 1986; Nagel and Huston, 1988) so that the facilitatory effects of biglycan and SP could be due to a reversal or attenuation of a lesion-induced deficit. However, the present data do not support this assumption since vehicle-controls displayed test latencies comparable to those found in sham-operated controls.

Unlike SP, biglycan did not influence preference behavior in the corral paradigm used to asses conditioned place preference/place aversion indicating that the proteoglycan has neither reinforcing or aversive properties. Therefore, it is rather unlikely that the memory enhancement of biglycan is the result of a "summation" of the noxious shock used to induce avoidance behavior plus aversive side effects of the treatment (Carey, 1987 for discussion). Further, the acute behavioral effects of biglycan and SP assessed during the conditioning trial in the closed Corral require attention. The behavioral pattern of rats that had received either SP (0.74 pmol) or biglycan in a dosage of 2.1 nmol did not differ from PBS-vehicle controls. Rats treated with 2.6 nmol biglycan showed a slight increase in locomotor activity and a tendency for reduced grooming behavior. These behavioral data show (a) that an increase or decrease in familiarity with the treatment environment secondary to a change in locomotor and/or exploratory activity cannot account for the conditioned place preference induced by SP and (b) provide further evidence for the assumption that both biglycan and SP did not facilitate retention of the uphill task by the way of proactive effects on test performance. Drugs that have been found to influence retention performance through proactive effects, e.g. muscimol, caused profound and long-lasting behavioral disturbances upon NBM injection (Nagel and Huston, 1988).

SEQUENCE LISTING

(1) GENERAL INFORMATION:

    (i) APPLICANT:
        (A) NAME: BOEHRINGER MANNHEIM GMBH
        (B) STREET: Sandhofer Str. 116
        (C) CITY: Mannheim
        (E) COUNTRY: Germany
        (F) POSTAL CODE (ZIP): D-68305
        (G) TELEPHONE: 08856/60-3446
        (H) TELEFAX: 08856/60-3451

   (ii) TITLE OF INVENTION: Use of chondroitin sulphate
       proteoglycans for protection of neurons

  (iii) NUMBER OF SEQUENCES: 2

   (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30B
            (EPO)

   (vi) PRIOR APPLICATION DATA:
        (A) APPLICATION NUMBER: WO PCT/EP94/01479
        (B) FILING DATE: 09-MAY-1994

(2) INFORMATION FOR SEQ ID NO: 1:

    (i) SEQUENCE CHARACTERISTICS:
        (A) LENGTH: 1107 base pairs
        (B) TYPE: nucleic acid
        (C) STRANDEDNESS: double
        (D) TOPOLOGY: linear

   (ii) MOLECULE TYPE: cDNA to mRNA

   (ix) FEATURE:
        (A) NAME/KEY: CDS
        (B) LOCATION:1..1107

   (ix) FEATURE:
        (A) NAME/KEY: sig_peptide
        (B) LOCATION:1..111

(ix) FEATURE:
        (A) NAME/KEY: mat_peptide
        (B) LOCATION:112..1107


        (xi) SEQUENCE DESCRIPTION: SEQ ID NO: 1:

```
ATG CGT CCC CTG TGG CTA CTC ACC TTG CTG CTG GCT CTG AGC CAG GCC      48
Met Arg Pro Leu Trp Leu Leu Thr Leu Leu Leu Ala Leu Ser Gln Ala
-37     -35                 -30                 -25

TTG CCC TTT GAG CAG AAG GGC TTC TGG GAC TTC ACC TTG GAT GAT GGG      96
Leu Pro Phe Glu Gln Lys Gly Phe Trp Asp Phe Thr Leu Asp Asp Gly
    -20                 -15                 -10

CTG CTC ATG ATG AAT GAT GAG GAG GCT TCA GGC TCA GAC ACC ACT TCA     144
Leu Leu Met Met Asn Asp Glu Glu Ala Ser Gly Ser Asp Thr Thr Ser
    -5                  1           5                   10

GGT GTC CCT GAC CTG GAC TCT CTC ACA CCT ACC TTC AGT GCC ATG TGT     192
Gly Val Pro Asp Leu Asp Ser Leu Thr Pro Thr Phe Ser Ala Met Cys
            15                  20                  25

CCT TTT GGC TGC CAC TGC CAC CTG CGG GTT GTT CAG TGC TCT GAC TTG     240
Pro Phe Gly Cys His Cys His Leu Arg Val Val Gln Cys Ser Asp Leu
            30                  35                  40

GGT CTG AAG ACT GTG CCC AAG GAG ATC TCA CCT GAT ACC ACA CTG CTA     288
Gly Leu Lys Thr Val Pro Lys Glu Ile Ser Pro Asp Thr Thr Leu Leu
    45                  50                  55

GAC CTG CAG AAC AAT GAC ATC TCT GAA CTC CGC AAG GAT GAC TTC AAA     336
Asp Leu Gln Asn Asn Asp Ile Ser Glu Leu Arg Lys Asp Asp Phe Lys
60                  65                  70                  75

GGC CTC CAG CAC CTC TAT GCT CTG GTC CTG GTA AAC AAT AAG ATC TCC     384
Gly Leu Gln His Leu Tyr Ala Leu Val Leu Val Asn Asn Lys Ile Ser
            80                  85                  90

AAG ATC CAT GAG AAG GCC TTT AGC CCT CTG CGG AAG CTG CAG AAA CTC     432
Lys Ile His Glu Lys Ala Phe Ser Pro Leu Arg Lys Leu Gln Lys Leu
            95                  100                 105

TAC ATC TCC AAG AAC CAC CTG GTG GAG ATT CCT CCC AAC CTG CCC AGC     480
Tyr Ile Ser Lys Asn His Leu Val Glu Ile Pro Pro Asn Leu Pro Ser
        110                 115                 120

TCC CTG GTA GAG CTA CGA ATC CAT GAC AAC CGT ATC CGC AAA GTG CCC     528
Ser Leu Val Glu Leu Arg Ile His Asp Asn Arg Ile Arg Lys Val Pro
        125                 130                 135
```

21

```
AAG GGC GTG TTC AGT GGG CTC CGG AAC ATG AAC TGC ATT GAG ATG GGT      576
Lys Gly Val Phe Ser Gly Leu Arg Asn Met Asn Cys Ile Glu Met Gly
140             145                 150                 155

GGG AAT CCC CTG GAG AAC AGT GGC TTT GAA CCC GGA GCC TTT GAT GGC      624
Gly Asn Pro Leu Glu Asn Ser Gly Phe Glu Pro Gly Ala Phe Asp Gly
                160                 165                 170

CTG AAG CTC AAC TAC CTG CGC ATC TCA GAG GCC AAG CTC ACT GGC ATC      672
Leu Lys Leu Asn Tyr Leu Arg Ile Ser Glu Ala Lys Leu Thr Gly Ile
            175                 180                 185

CCC AAA GAT CTC CCT GAG ACC CTG AAT GAA CTT CAC TTG GAC CAC AAC      720
Pro Lys Asp Leu Pro Glu Thr Leu Asn Glu Leu His Leu Asp His Asn
            190                 195                 200

AAA ATC CAG GCT ATC GAG TTG GAG GAT CTA CTT CGC TAC TCC AAG CTG      768
Lys Ile Gln Ala Ile Glu Leu Glu Asp Leu Leu Arg Tyr Ser Lys Leu
            205                 210                 215

TAC AGG CTG GGC TTA GGA CAC AAT CAG ATC CGA ATG ATT GAG AAT GGG      816
Tyr Arg Leu Gly Leu Gly His Asn Gln Ile Arg Met Ile Glu Asn Gly
220             225                 230                 235

AGC CTG AGT TTT CTG CCT ACC CTG AGG GAA CTT CAC TTG GAC AAC AAC      864
Ser Leu Ser Phe Leu Pro Thr Leu Arg Glu Leu His Leu Asp Asn Asn
                240                 245                 250

AAG CTG TCC CGG GTG CCT GCT GGC CTC CCA GAT CTC AAA CTC CTC CAG      912
Lys Leu Ser Arg Val Pro Ala Gly Leu Pro Asp Leu Lys Leu Leu Gln
                255                 260                 265

GTT GTC TAT CTG CAC TCC AAC AAC ATC ACC AAG GTG GGC ATC AAC GAT      960
Val Val Tyr Leu His Ser Asn Asn Ile Thr Lys Val Gly Ile Asn Asp
            270                 275                 280

TTC TGC CCC ATG GGC TTT GGA GTC AAG AGG GCC TAC TAT AAT GGC ATC     1008
Phe Cys Pro Met Gly Phe Gly Val Lys Arg Ala Tyr Tyr Asn Gly Ile
            285                 290                 295

AGC CTC TTC AAC AAC CCT GTG CCC TAC TGG GAA GTG CAG CCT GCC ACC     1056
Ser Leu Phe Asn Asn Pro Val Pro Tyr Trp Glu Val Gln Pro Ala Thr
300                 305                 310                 315

TTC CGC TGC GTC ACT GAC CGC CTG GCC ATC CAA TTT GGA AAT TAT AAG     1104
Phe Arg Cys Val Thr Asp Arg Leu Ala Ile Gln Phe Gly Asn Tyr Lys
                320                 325                 330

AAG                                                                 1107
Lys
```

22

(2) INFORMATION FOR SEQ ID NO: 2:

    (i) SEQUENCE CHARACTERISTICS:
       (A) LENGTH: 369 amino acids
       (B) TYPE: amino acid
       (D) TOPOLOGY: linear

    (ii) MOLECULE TYPE: protein
    (xi) SEQUENCE DESCRIPTION: SEQ ID NO: 2:

```
Met Arg Pro Leu Trp Leu Leu Thr Leu Leu Leu Ala Leu Ser Gln Ala
-37     -35             -30             -25

Leu Pro Phe Glu Gln Lys Gly Phe Trp Asp Phe Thr Leu Asp Asp Gly
    -20             -15             -10

Leu Leu Met Met Asn Asp Glu Glu Ala Ser Gly Ser Asp Thr Thr Ser
 -5             1             5             10

Gly Val Pro Asp Leu Asp Ser Leu Thr Pro Thr Phe Ser Ala Met Cys
            15             20             25

Pro Phe Gly Cys His Cys His Leu Arg Val Val Gln Cys Ser Asp Leu
        30             35             40

Gly Leu Lys Thr Val Pro Lys Glu Ile Ser Pro Asp Thr Thr Leu Leu
    45             50             55

Asp Leu Gln Asn Asn Asp Ile Ser Glu Leu Arg Lys Asp Asp Phe Lys
 60             65             70             75

Gly Leu Gln His Leu Tyr Ala Leu Val Leu Val Asn Asn Lys Ile Ser
            80             85             90

Lys Ile His Glu Lys Ala Phe Ser Pro Leu Arg Lys Leu Gln Lys Leu
            95             100            105

Tyr Ile Ser Lys Asn His Leu Val Glu Ile Pro Pro Asn Leu Pro Ser
        110            115            120

Ser Leu Val Glu Leu Arg Ile His Asp Asn Arg Ile Arg Lys Val Pro
    125            130            135

Lys Gly Val Phe Ser Gly Leu Arg Asn Met Asn Cys Ile Glu Met Gly
140            145            150            155

Gly Asn Pro Leu Glu Asn Ser Gly Phe Glu Pro Gly Ala Phe Asp Gly
            160            165            170

Leu Lys Leu Asn Tyr Leu Arg Ile Ser Glu Ala Lys Leu Thr Gly Ile
            175            180            185
```

```
Pro Lys Asp Leu Pro Glu Thr Leu Asn Glu Leu His Leu Asp His Asn
        190                 195                 200

Lys Ile Gln Ala Ile Glu Leu Glu Asp Leu Leu Arg Tyr Ser Lys Leu
        205                 210                 215

Tyr Arg Leu Gly Leu Gly His Asn Gln Ile Arg Met Ile Glu Asn Gly
220                 225                 230                 235

Ser Leu Ser Phe Leu Pro Thr Leu Arg Glu Leu His Leu Asp Asn Asn
            240                 245                 250

Lys Leu Ser Arg Val Pro Ala Gly Leu Pro Asp Leu Lys Leu Leu Gln
            255                 260                 265

Val Val Tyr Leu His Ser Asn Asn Ile Thr Lys Val Gly Ile Asn Asp
        270                 275                 280

Phe Cys Pro Met Gly Phe Gly Val Lys Arg Ala Tyr Tyr Asn Gly Ile
        285                 290                 295

Ser Leu Phe Asn Asn Pro Val Pro Tyr Trp Glu Val Gln Pro Ala Thr
300                 305                 310                 315

Phe Arg Cys Val Thr Asp Arg Leu Ala Ile Gln Phe Gly Asn Tyr Lys
            320                 325                 330

Lys
```

## Claims

1. Use of a proteoglycan with a part or all of the primary amino acid structure of SEQ ID NO:1 and at least one attached glycosaminoglycan chain for promotion of neurite outgrowth and/or support of survival of neurons in vivo and in vitro.

2. Use according to claim 1, wherein the proteoglycan is mammalian biglycan.

3. Use according to claim 2, wherein the biglycan is represented by the amino acid sequence SEQ ID NO:1 with the variations of Figure 8.

4. Use of a proteoglycan with a part or all of the primary amino acid structure of SEQ ID NO:1 and at least with one attached glycosaminoglycan chain for the manufacturing of a therapeutic agent for the prevention of neural cell degeneration and/or for regeneration of the nervous system.

5. Use according to claim 4, wherein the proteoglycan is mammalian biglycan.

6. Use according to claim 5, wherein the biglycan is represented by the amino acid sequences SEQ ID NO:1 with the variations of Figure 8.

7. Use of a proteoglycan according to claims 4 to 6 for improving the learning efficiency and the memory.

8. Method of production of a proteoglycan with a part or all of the primary amino acid structure of SEQ ID NO:1, by recombinant methods in prokaryotes, wherein the DNA sequence of said proteoglycan is cloned in a vector and transformed in a prokaryotic host cell for replication, the host cell is cultivated under conditions for the amplification of the vector, the desired protein is isolated after fermentation,

EP 0 686 397 A2

one or more glycosaminoglycan side chains are attached by chemical or enzymatic coupling of glucosides to said protein via activated groups of the protein and the glycosylated protein is isolated.

9. The method according to claim 8, wherein the proteoglycan is mammalian biglycan.

10. The method according to claim 9, wherein the biglycan is represented by the amino acid sequence SEQ ID NO:1 with the variations of Figure 8.

11. Proteoglycan with a part or all of the primary amino acid structure of SEQ ID NO:1, which differs from naturally occurring biglycan in glycosylation pattern and/or amino acid sequence, which is obtainable by recombinant methods and wherein the DNA sequence of said proteoglycan is cloned in a vector and transformed in a host cell for replication, the host cell is cultivated under conditions for the amplification of the vector, the desired protein is isolated after fermentation.

12. Proteoglycan according to claim 11, wherein the vector is transferred in a prokaryotic host cell and after fermentation one or more glycosaminoglycan side chains are attached by chemical or enzymatic coupling of glucosides to said protein via activated groups of the protein and the glycosylated protein is isolated.

25

# Fig. 1

A          B

C          D

E

OD 550 nm/1000

micregram protein/well

**Fig. 2**

**Fig. 3**

**Fig. 4**

27

Fig. 5

# Fig. 6

# Fig. 7

# Fig. 8

```
      -37                              .                    .                    .            +1                                     23
        |                                                                               |                    ┌─────────────────────────────┐
RAT     MRPLWLLTLLLALSQALPFEQKGFWDFTLDDGLLMMNDEEASG│SDTTSGVPDLDSLTPTF
HUMAN     W   R VS                    R              PF              │A *        L P    V      Y
BOVINE  ------------------------------------------- │AE         I         P      Y
                                                      └─────────────────────────────┘

                                                                                        83
                 .                    .                    .                    .         |
        SAMCPFGCHCHLRVVQCSDLGLKTVPKEISPDTTLLDLQNNDISELRKDDFKGLQHLYAL
                                       S
                                       A

                                                                                       143
                 .                    .                    .                    .         |
        VLVNNKISKIHEKAFSPLRKLQKLYISKNHLVEIPPNLPSSLVELRIHDNRIRKVPKGVF

                                                                                       203
                 .                    .                    .                    .         |
        SGLRNMNCIEMGGNPLENSGFEPGAFDGLKLNYLRISEAKLTGIPKDLPETLNELHLDHN
          E

                                                                                       263
                 .                    .                    .                    .         |
        KIQAIELEDLLRYSKLYRLGLGHNQIRMIENGSLSFLPTLRELHLDNNKLSRVPAGLPDL
                                                                        A       S

                                                                                       323
                 .                    .                    .                    .         |
        KLLQVVYLHSNNITKVGINDFCPMGFGVKRAYYNGISLFNNPVPYWEVQPATFRCVTDRL
                           V
             T             V           V

        AIQFGNYKK

             Y*
```

# Fig. 9

UPHILL AVOIDANCE

# Fig. 10

**UPHILL AVOIDANCE**

BIGLYCAN

# Fig. 11

**CONDITIONED CORRAL PREFERENCE**